# EUROPÄISCHE PATENTSCHRIFT

(11) **EP 1 529 501 B1**
(45) Veröffentlichungstag und Bekanntmachung des Hinweises auf die Patenterteilung: **12.07.2006**
(21) Anmeldenummer: 04021422.3
(22) Anmeldetag: 09.09.2004
(51) Int. Cl.: A61F 2/78, A61F 2/28

(54) **Subkutanes, intramuskuläres Lager für ein starres transkutanes Implantat**
Subcutaneous, intra-muscular coupling for a rigid transcutaneous implant
Accouplement intramusculairé sous-cutané pour un implant transcutané fixe

(30) Priorität: 07.11.2003 DE 10353400; 18.02.2004 DE 102004008558
(43) Veröffentlichungstag der Anmeldung: 11.05.2005
(73) Patentinhaber: ESKA Implants GmbH & Co., 23556 Lübeck (DE)
(72) Erfinder: Grundei, Hans, Dr.-Ing., 23558 Lübeck (DE)
(74) Vertreter: Fuchs Mehler Weiss & Fritzsche

(56) Entgegenhaltungen:
- EP-A- 1 378 215
- EP-A- 1 407 727
- DE-A- 10 040 590
- US-A- 4 158 895

## Beschreibung

Die Erfindung betrifft ein subkutanes, intramuskuläres Lager für ein starres transkutanes Implantat, welches intrakorporal in einem Knochenstumpf verankerbar ist und welches ein Zwischenstück zwischen dem Implantat und einer daran ankoppelbaren extrakorporalen Kopplungseinrichtung aufweist.

Ein derartiges Lager ist bekannt aus der DE 100 40 590 A1. Das darin beschriebene Lager besteht aus einem flexiblen Material und es weist eine Tülle auf, die das Implantat distal fest umschließt, sowie eine intrakorporal anzuordnende Überwurfhülse in Form eines flexiblen Faltenbalges, der proximal mit einem angeformten Bund in abdichtender Weise mit der Tülle verbunden ist, derart, dass zwischen der Innenwandung des Faltenbalges und Außenwandung der Tülle ein Hohlraum einer Mindestbreite frei bleibt. Dabei ist distal am Faltenbalg ein flexibles Gitternetzwerk angeordnet, dem sich distalseitig ein weiteres Gitternetzwerk mit einem höheren E-Modul anschließt.

Mit diesem Lager wird das Ziel verfolgt, dass sich Weichteile gegenüber dem starren Implantat bewegen können, ohne dass die Durchbruchstelle im Körperstumpfteil einem erhöhten Risiko einer Entzündung ausgesetzt wird.

Wenn dieses bekannte Lager auch bereits in der Praxis erfolgreich eingesetzt wird, birgt es das Risiko, dass im Falle beispielsweise einer Reinigung der Durchtrittstelle des Implantates durch den Ober schenkelstumpf mit einer Kanüle durch das flexible Material, in den meisten Fällen Silikon, hindurchgestochen wird und eine Verkeimung stattfindet.

Vor diesem Hintergrund ist es nun die Aufgabe der vorliegenden Erfindung, ein gattungsgemäßes, subkutanes intramuskuläres Lager der eingangs genannten Art so weiterzubilden, dass die Sicherheit gegen eine Verkeimung der Durchtrittsstelle des Implantates und der angrenzenden Bereiche des Oberschenkelstumpfes deutlich erhöht wird und dass ein versehentliches Entfernen der Keimschranke verhindert wird.

Demgemäß wird vorgeschlagen, dass das Lager gemäß einem ersten Vorschlag eine mit dem Zwischenstück fest verbundene starre Buchse derart aufweist, dass zwischen der Wandung der Buchse und dem Zwischenstück ein in Richtung intrakorporal geschlossener Ringraum ausgebildet ist, in den die extrakorporale Kopplungseinrichtung setzbar ist. Auf der Außenwandung der Buchse ist eine offenmaschige, dreidimensionale Raumnetzstruktur vorgesehen, allerdings unter Aussparung im distalen Bereich um eine Breite B, vorzugsweise bis zu 2cm. Des Weiteren ist ein Federring vorgesehen, der von distal in den Ringraum setzbar, dort teleskopartig verschiebbar und unter Ausübung seiner Federkraft arretierbar ist.

Gegenüber dem bekannten Lager ist die Buchse vorliegend ein starres Element, das nicht etwa durch Injektionskanülen durchdrungen werden kann. Die auf der Außenwandung der Buchse vorgesehene offenmaschige, dreidimensionale Raumnetzstruktur dient dazu, dass sich Bindegewebe darin einorganisieren kann und so eine Keimsperre ausbildet. Der distale Bereich bleibt dabei von der Raumnetzstruktur ausgespart, um so eine Bewegung des umgebenden Bindegewebes bei Ausgleichbewegungen zu ermöglichen.

Der Federring wird beispielsweise mit einer Spannzange zusammengedrückt und in den Ringraum eingesetzt und darin so verschoben, dass die Schicht von Bindegewebe ab dem Ende der Raumnetzstruktur auf der Buchse bis hin zur Haut des Oberschenkelstumpfes überbrückt wird. Die Einstellbarkeit richtet sich nach patientenindividuellen Gegebenheiten.

Operativ geht man nun so vor, dass nach der Amputation des Oberschenkels zunächst das transkutane Implantat im Knochenstumpf verankert wird und distalseitig mit dem Zwischenstück versehen wird. Daraufhin wird der Oberschenkelstumpf verschlossen, solange, bis das transkutane Implantat im Knochenstumpf verwachsen ist. Sodann, nach etwa 6 bis 8 Wochen, wird der Oberschenkelstumpf mit einer Hautstanze eröffnet und der Federring mittels einer Spannzange so zusammengedrückt, dass er in den Ringraum geführt werden kann.

Der Federring ist vorzugsweise ein abgewinkelter Ring mit einem Radialschlitz. Der abgewinkelte Flansch legt sich dann von außen gegen die Haut des Oberschenkelstumpfes. Der Radialschlitz dient zweierlei Zwecken: Zum einen kann der Federring zum Einsetzen in den Ringraum zusammengedrückt werden. Nach Entfernen der Spannzange dehnt sich der Ring im Umfang aus und erzeugt dadurch die für die Arretierung notwendige Federkraft. Darüber hinaus dient der Radialschlitz zum Abfluss bzw. Abführen körpereigener Sekrete.

Die Maschenweiten der dreidimensionalen Raumnetzstruktur auf der Buchse betragen vorzugsweise 50µm bis 2500µm. Diese Maschenweiten sind relativ groß, nehmen das sie umgebende Bindegewebe jedoch in hinreichendem Maße auf, damit eine sichere Keimsperre entstehen kann.

Die Buchse kann durch Aufschrumpfen auf das Zwischenstück fest mit diesem verbunden sein. Alternativ kann sie mit diesem auch verschweißt sein. Es ist auch möglich, dass die Buchse mit dem Zwischenstück einstückig ausgebildet ist. In jedem Falle muss gewährleistet sein, dass die Verbindung zwischen der Buchse und dem Lager fest und starr ist und den auftretenden Belastungen widerstehen kann.

Ein zweiter Vorschlag sieht ein subkutanes Lager vor, mit einer mit dem Zwischenstück fest verbundenen starren Buchse mit einem in Richtung intrakorporal geschlossenen Ankopplungselement, an das die extrakorporale Kopplungseinrichtung koppelbar ist, mit einer offenmaschigen dreidimensionalen Raumnetzstruktur auf der Außenwandung der Buchse unter Aussparung im distalen Bereich um eine Breite B, mit einer im Bereich des Durchtritts durch den Beinstumpf vorgesehenen aktivierbaren Vorrichtung zur Applikation bioaktiven Materials, und mit einem Federring, der von distal in das Ankopplungselement setzbar ist, teleskopartig verschiebbar und über Ausübung seiner Federwirkung arretierbar ist.

Das Lager gemäß diesem zweiten Vorschlag unterscheidet sich von jenem gemäß dem ersten Vorschlag im wesentlichen also durch die im Bereich des Durchtritts durch den Beinstumpf vorgesehene aktivierbare Vorrichtung zur Applikation bioaktiven Materials. Hierdurch ist eine optimale Versorgung der Durchtrittsstelle durch den Oberschenkelstumpf gewährleistet. Unter dem Begriff des bioaktiven Materials wird dabei ein Medikament verstanden.

Der kritische Bereich des Durchtritts des Implantates durch den Oberschenkelstumpf kann also medikamentös versorgt werden im Hinblick auf eine verbesserte Wundheilung, aber auch im Hinblick auf eine Prophylaxe gegen Entzündungen. Die Wundheilung wird durch die Applikation eines Medikamentes stark verbessert.

Gemäß einer ersten bevorzugten Ausführungsform dieses zweiten Vorschlags ist vorgesehen, dass die aktivierbare Vorrichtung zur Applikation bioaktiven Materials mindestens eine in die Außenwandung der Buchse eingebrachte umlaufende Ringnut und mindestens einen in die Ringnut setzbaren Hohlring aus elastischem und porösem Material mit einem angeformten Zulauf, durch welchen der Hohlring mit flüssigen bioaktiven Material beschickt werden kann, aufweist. Durch den Zulauf wird das bioaktive Material in das Innere des Hohlringes verbracht. Aus diesem tritt das Material aufgrund der Porosität dann im Laufe der Zeit aus und benetzt die darunter liegenden Bereiche der offenmaschigen Raumnetzstruktur und fließt dann in Richtung des Durchtritts des Implantates durch den Oberschenkelstumpf. Der Hohlring stellt ein gewisses Reservoir dar. Die Speicherfähigkeit in zeitlicher Hinsicht wird maßgeblich beeinflusst durch die Porosität des Materials, aus welchem er besteht. Vorzugsweise wird diese so gewählt, dass bioaktives Material lediglich etwa jede Woche zugeführt werden muss.

In besonders bevorzugter Weiterbildung bestehen der Hohlring und der Zulauf aus Silikon. Dabei sind der Hohlring und der Zulauf vorzugsweise einstückig ausgebildet.

Gemäß einer zweiten bevorzugten Ausführungsform des zweiten Vorschlags umfasst die Vorrichtung zur Applikation des bioaktiven Materials mindestens eine in die Außenwandung der Buchse eingebrachte umlaufende Ringnut und mindestens einen seitlich neben dem Ankoppelungselement angeordneten Stichkanal in der Buchse, der so verläuft, dass er die mindestens eine Ringnut peripher schneidet. Er dient zum Einbringen des bioaktiven Materials. Nach der Applikation des bioaktiven Materials kann der Stichkanal besonders bevorzugt durch einen Verschluss, beispielsweise durch eine Schraube, wieder verschlossen werden. Bei dieser Ausführungsform kommt es also weniger auf die Langzeitapplikation des bioaktiven Materials als vielmehr auf die spontane und kurzfristige Versorgung des Durchtritts des Implantates durch den Oberschenkelstumpf an.

In besonders bevorzugter Weiterbildung dieser Ausführungsform ist vorgesehen, dass auf der Außenwandung der Buchse im unteren Bereich der Raumnetzstruktur zwischen der untersten Ringnut und dem raumnetzstrukturfreien Bereich der Buchse mindestens ein Stichkanal, besonders bevorzugt drei Stichkanäle, verlaufen zur Weiterleitung des bioaktiven Materials hin zum Durchtritt des Beinstumpfes.

Gemäß einer dritten konkreten bevorzugten Ausführungsform des zweiten Vorschlags ist vorgesehen, dass um das Ankopplungselement herum ein umlaufender Ringraum angeordnet ist, in welchem ein Vorrat eines elastischen Filmes aus bioaktivem Material gelagert ist. Der Film tritt dabei aus dem Ringraum durch einen ringförmigen Schlitz in der Buchse aus und verläuft entlang des raumnetzstrukturfreien Bereiches der Buchse bis zum Durchtritt durch den Beinstumpf. Dabei umhüllt er die Hülse schlauchförmig. In dem raumnetzstrukturfreien Bereich haftet der Film an dem ihn umgebenden Gewebe bzw. Haut. Aufgrund des Wachstums der Haut und des Gewebes wird der Film auf diese Weise nach außen gezogen, wobei er eventuelle Keime und Bakterien mit sich führt. Das Wachstum des Gewebes und der Haut beträgt maximal ca. 1 mm pro Tag. Dementsprechend wird der Film um dieses Maß aus dem Zwischenstück gezogen. Standzeiten von bis zu einem Jahr des Vorrates des Filmes sind möglich, bevor der Ringraum mit einem neuen Vorrat an Film beschickt werden muss.

Diese Ausführungsform zielt nicht ab auf eine spontane kurzfristige Wundheilbehandlung. Vielmehr steht hier die langfristige Prophylaxe gegen eine etwaige Verkeimung im Vordergrund.

Alle Ausführungsformen des Lagers gemäß dem zweiten Vorschlag können noch vorteilhaft dadurch weitergebildet sein, dass das Ankopplungselement als konische Klemmhülse ausgebildet ist. Dies gestattet eine kleinere Baugröße als ein Doppelkonus, wie er im Rahmen des ersten Vorschlags beschrieben worden ist. Im übrigen können alle Weiterbildungen des ersten Vorschlags Anwendung finden auf den zweiten Vorschlag.

Die Erfindung wird anhand von Ausführungsbeispielen näher erläutert. Hierbei zeigt:
- Fig. 1: das subkutane, intramuskuläre Lager, angekoppelt an einem transkutanen Implantat gemäß einem ersten Ausführungsbeispiel,
- Fig. 2: eine Ausführungsform des Federringes,
- Fig. 3: schematisch das Zwischenstück des Lagers mit Buchse und Federring,
- Fig. 4: schematisch das transkutane Implantat mit dem Zwischenstück gemäß einem zweiten Ausführungsbeispiel,
- Fig. 5: das Zwischenstück in Vergrößerung,
- Fig. 6: den Hohlring in Aufsicht (a) und in Seitenansicht (b),
- Fig. 7: das Zwischenstück gemäß der zweiten Ausführungsform in schematischer Schnittansicht (a) und in schematischer Ansicht (b), und
- Fig. 8: das Zwischenstück gemäß einer dritten Ausführungsform in Schnittansicht.

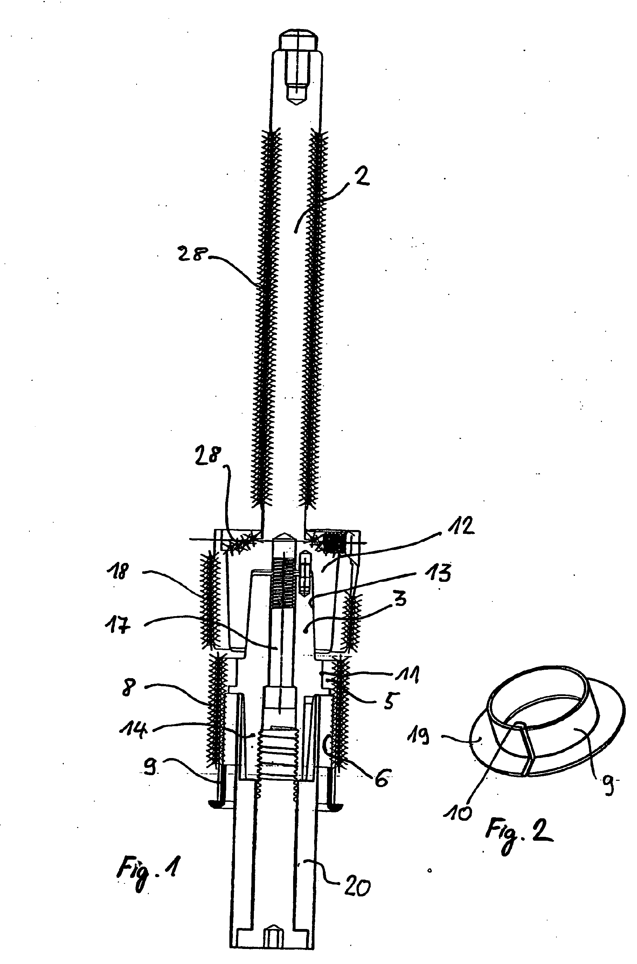

Einen ersten Überblick verschafft Fig. 1. Ein starres transkutanes Implantat 2 wird in einen Femurstumpf (nicht dargestellt) eingesetzt. Die offenmaschige, dreidimensionale Raumnetzstruktur 28 dient im vorliegenden Fall zum Einorganisieren von Knochenmaterial zur Sekundärfixation des Implantates 2 im Knochen. Es ist distalseitig abgeschlossen durch eine Metallhülse 12, welche den Femurstumpf abschließt. Hierzu trägt die Metallhülse 12 auch eine dreidimensionale, offenmaschige Raumnetzstruktur 28, in welche Knochenmaterial einwachsen soll.

Im Inneren der Metallhülse 12 ist eine konische Klemmhülse 13 ausgebildet. Diese ist vorgesehen zur Herstellung einer konischen Klemmverbindung mit dem vorliegend als Doppelkonus ausgebildeten Zwischenstück 3. Das Zwischenstück 3 weist einen zylindrischen Mittelabschnitt 11 auf, auf dem die Buchse 5 vorliegend aufgeschrumpft ist. Dem Mittelabschnitt 11 schließt sich distalseitig ein weiterer Konus 14 zur Herstellung einer konischen Verklemmung mit einer konischen Klemmhülse in einem Adapter der extrakorporalen Koppelungseinrichtung (nicht dargestellt) an.

Die Buchse 5 ist so ausgebildet, dass zwischen ihrer Wandung und dem Zwischenstück 3 ein in Richtung intrakorporal oder proximal geschlossener Ringraum 6 ausgebildet ist. In diesen Ringraum 6 wird die extrakorporale Kopplungseinrichtung gesetzt.

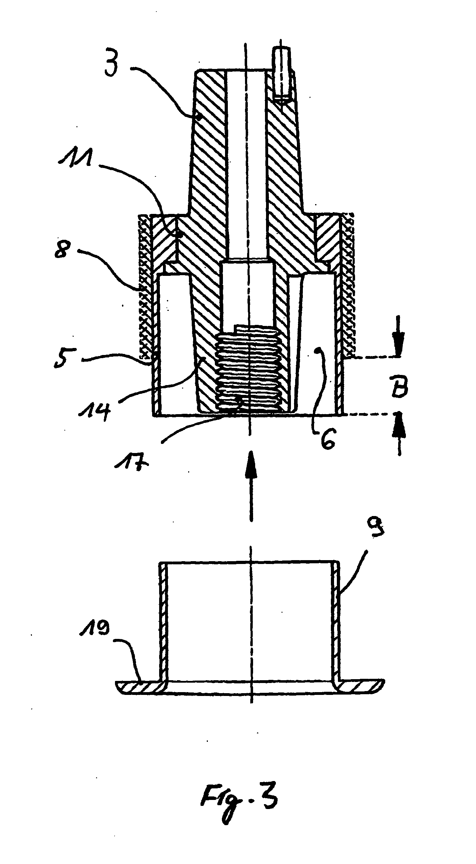

Die Außenseite der Buchse 5 weist im proximalen Bereich eine offenmaschige, dreidimensionale Raumnetzstruktur 8 auf, in welche das nach Implantation die Buchse 5 umgebende Bindegewebe eingranuliert, um so eine Keimsperre auszubilden. Im distalen Bereich der Außenwandung der Buchse 5 befindet sich in einem Bereich der Breite B (Fig. 3) keine Raumnetzstruktur. Dies gestattet eine Ausgleichsbewegung des umgebenden Bindegewebes, ohne dass es zu Spannungen im Gewebe kommt.

Bei der Implantation wird zunächst das transkutane Implantat 2 in den Femurstumpf mit daran angebrachter Metallhülse 12 implantiert und der Oberschenkelstumpf dann zur Einheilung des Implantates verschlossen. Nach 6 bis 8 Wochen ist genügend Knochenmaterial in die Raumnetzstruktur 28 des Implantates 2 eingewachsen, so dass dieses stabil auch unter Belastungen im Femurstumpf verbleibt. Gleichzeitig ist Bindegewebe in die Raumnetzstruktur 18 an der Außenwandung der Metallhülse 12 eingewachsen, um eine erste Keimsperre auszubilden.

Nach der erwähnten Zeitspanne wird der Oberschenkelstumpf wieder eröffnet und das Zwischenstück 3 mit der Buchse 5 wird in die Öffnung im Femurstumpf geführt und mittels konischer Klemmverbindung zwischen dem Doppelkonus und der Klemmhülse 13 dort arretiert. Ein zusätzliches Sicherungsmittel 17 (hier in Form einer Schraube) dient zur zusätzlichen Sicherung.

Nach Eröffnung des Oberschenkelstumpfes wird der Federring 9 (Fig. 2) mittels einer Spannzange zusammengedrückt, wozu der Radialschlitz 10 vorgesehen ist und in den Ringraum 6 eingeführt. Durch teleskopartiges Einführen oder Verschieben des Ringes 9 in den Ringraum kann der patientenindividuelle Abstand zwischen dem distalen Ende der Buchse 5 und der Haut des Oberschenkelstumpfes eingestellt werden. Der abgewinkelte Flansch 19 des Federringes 9 legt sich dann an die Haut des Oberschenkelstumpfes an. Der Schlitz 10 dient dabei zum einen der Möglichkeit, den Federring mit einer Spannzange zusammen zu drücken, um so beim Lösen der Spannzange eine Federkraft zu erzeugen und zum anderen zum Ablauf eines etwaigen körpereigenen Sekretes.

In den Ringraum 6 eingeführt ist vorliegend ein Messbolzen 20, mit dessen Hilfe die erforderliche Länge für die extrakorporale Koppelungseinrichtung ermittelt werden kann.

Die Verhältnisse werden vergrößert nochmals aus Fig. 3 deutlich. Hier ist isoliert dargestellt das Zwischenstück 3 mit aufgeschrumpfter Buchse 5, so dass sich ein Ringraum 6 zwischen dem Konus 14 und der Buchse 5 ergibt.

Das Aufschrumpfen der Buchse 5 auf das Zwischenstück 3 gelingt vorliegend besonders gut aufgrund des zylindrischen Mittelabschnittes 11 des Zwischenstückes 3. Deutlich erkennbar ist auch die offenmaschige, dreidimensionale Raumnetzstruktur 8 auf der Außenwandung der Buchse 5, in welche das sie nach Implantation umgebende Bindegewebe einwächst zur Bildung der Keimsperre. Eingezeichnet ist in Fig. 3 auch die Breite B, also die Breite des Bereiches, der distalseitig frei von einer Raumnetzstruktur 8 ist. Aus Fig. 3 ist auch aufgrund des angedeuteten Pfeils ersichtlich, wie der Klemmring 9 in den Ringraum 6 eingeführt wird. Nach Auffinden der richtigen Einführungstiefe wird dann die Spannzange gelöst und der Federring 9 spreizt sich so, dass er sich gegen die Innenwandung der Buchse 5 legt und in dieser Stellung verharrt.

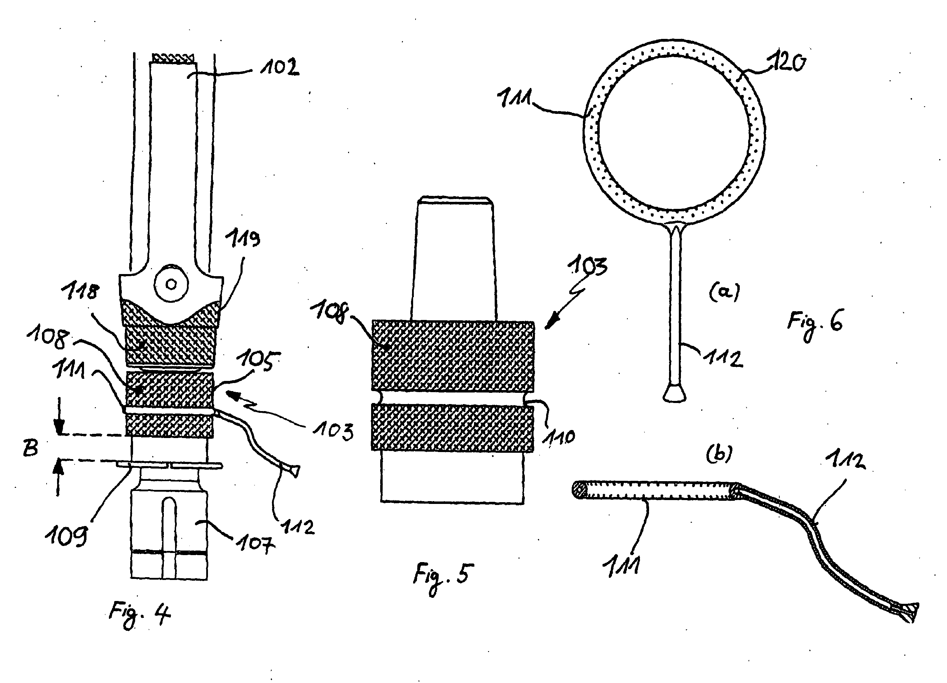

Figur 4 zeigt nochmals das starre transkutane Implantat 102, das intrakorporal in einen Knochenstumpf verankerbar ist. Es weist wieder ein Zwischenstück 103 zwischen dem Implantat 102 und einer daran ankoppelbaren extrakorporalen Kopplungseinrichtung 107 auf.

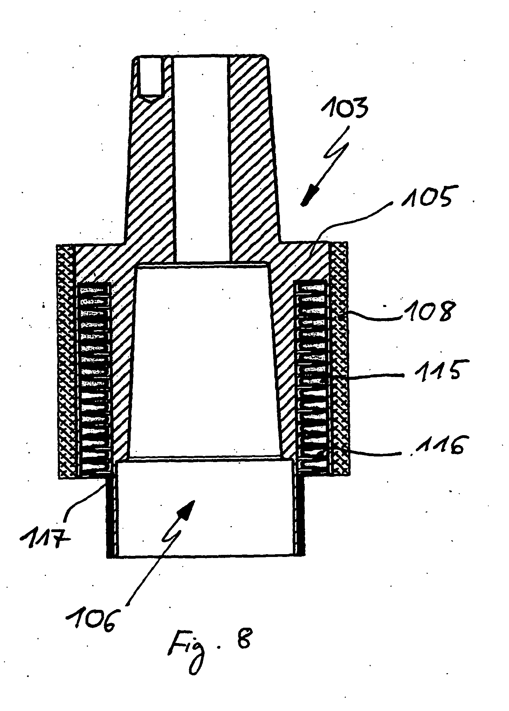

Mit dem Zwischenstück 103 ist eine starre Buchse 105 verbunden. Die Buchse 105 weist einen in Richtung intrakorporal geschlossenes Ankopplungselement 106 (Figuren 7a und 8) auf, an welches die extrakorporale Kopplungsvorrichtung 107 gekoppelt ist.

Die Außenwandung der Buchse 105 trägt wieder eine offenmaschige dreidimensionale Raumnetzstruktur 108, in welche Bindegewebe eingranuliert, um so eine Keimsperre zu bilden. Die das Implantat 102 abschließende Metallhülse 119 trägt gleichfalls die dreidimensionale offenmaschige Raumnetzstruktur 118 zu demselben Zweck.

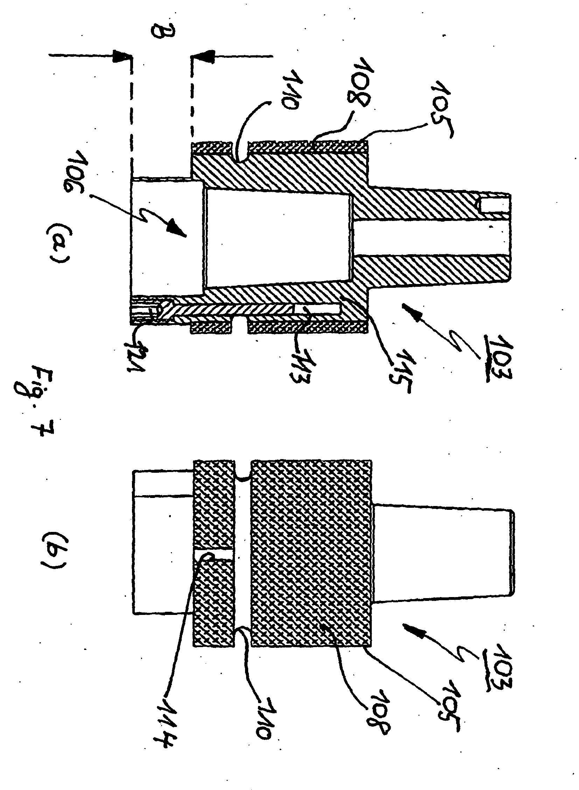

In die Außenwandung der Buchse 105 ist im dargestellten Ausführungsbeispiel eine umlaufende Ringnut 110 eingelassen. In diese Ringnut 110 ist ein Hohlring 111 mit angeschlossenem Zulauf 112 eingelegt. Einzelheiten des Hohlringes 111 sind aus Figur 6 ersichtlich.

Der Zulauf 112 ist direkt am Hohlring 111 angeformt. Beide bestehen vorzugsweise aus Silikon. Der Hohlring 111 ist porös oder weist kleine Löcher 120 auf, aus welchen das eingebrachte bioaktive Material austreten kann und somit seine therapeutische Wirkung im Bereich des Durchtritts des Implantates durch den Oberschenkelstumpf entfalten kann.

Figur 7 zeigt nun eine zweite Ausführungsform des Zwischenstücks 103. Die wieder mit der Raumnetzstruktur 108 belegte Buchse 105 weist vorliegend eine umlaufende Ringnut 110 auf. Wie man an der Schnittansicht (Fig. 7a) erkennen kann, ist in der rechten Seite der Buchse 105 ein Stichkanal 113 vorgesehen, der vorliegend mit einer eingedrehten Schraube 121 verschlossen ist. Der Stichkanal 113 ist so in die Buchse 105 eingebracht, dass er die Ringnut 110 peripher schneidet, so dass ein in den Stichkanal 113 eingebrachtes bioaktives Material aus dem Stichkanal 113 in die Ringnut 110 treten kann, um dort seine therapeutische Wirkung auszuüben. Zum Einbringen des bioaktiven Materials muss die Schraube 121 aus dem Stichkanal 113 herausgeschraubt werden, wonach dann beispielsweise mit einer Kanüle das bioaktive Material in den Kanal 113 gespritzt werden kann. Nach erfolgter Behandlung wird der Stichkanal 113 wieder mit der Schraube 121 verschlossen.

Damit eine gute Verteilung des bioaktiven Materials vonstatten gehen kann, sind vorliegend drei Stichkanäle 114 (Fig. 7b) vorgesehen, welche die Ringnut 110 mit dem raumnetzstrukturfreien Bereich der Buchse 105 der Breite B verbindet. Das bioaktive Material fließt dann aus der Ringnut 110 über die Stichkanäle 114 in Richtung der Durchtrittsstelle des Implantats.

Figur 8 zeigt schließlich die dritte bevorzugte Ausführungsform des Zwischenstückes 103. Vorliegend ist um das Ankopplungselement 106 ein umlaufender Ringraum 115 ausgebildet. In dem Ringraum 115 befindet sich ein Vorrat eines elastischen Filmes 116 aus bioaktivem Material.

Der Film in Form eines zusammengestauchten Schlauches tritt durch einen ringförmigen Schlitz 117 aus dem Ringraum 115 heraus und verläuft dann entlang des raumnetzstrukturfreien Bereichs der Buchse 105 bis zum Durchtritt durch den Beinstumpf. Der Film 116 umhüllt dabei diesen Abschnitt der Hülse 105 schlauchförmig. Im unteren Bereich haftet der Film 116 an das umgebende Gewebe bzw. Haut und wird mit Wachstum der Haut und des Gewebes durch den Durchtrittsbereich des Implantates nach außen gezogen. Dabei nimmt der Film etwaige Keime mit nach außen. Der Patient kann dann von Zeit zu Zeit das ausgetretene Filmmaterial 116 abschneiden.

### Bezugszeichenliste

- 2: traskutanes Implantat
- 3: Zwischenstück

- 5: Buchse
- 6: Ringraum

- 8: Raumnetzstruktur
- 9: Federring
- 10: Radialschlitz
- 11: zylindrischer Mittelabschnitt
- 12: Metallhülse
- 13: konische Klemmhülse
- 14: Konus

- 17: Sicherungsmittel

- 19: Flansch
- 20: Meßbolzen

- 28: Raumnetzstruktur

- 102: transkutanes Implantat
- 103: Zwischenstück

- 105: Buchse
- 106: Ankopplungselement
- 107: Kopplungseinrichtung
- 108: Raumnetzstruktur
- 109: Federring
- 110: Ringnut
- 111: Hohlring
- 112: Zulauf
- 113: Stichkanal
- 114: Stichkanal
- 115: Ringraum
- 116: Film
- 117: Schlitz
- 118: Raumnetzstruktur
- 119: Metallhülse
- 120: Locher
- 121: Schraube

## Patentansprüche

1. Subkutanes, intramuskuläres Lager (1) für ein starres transkutanes Implantat (2), welches intrakorporal in einem Knochenstumpf verankerbar ist und welches ein Zwischenstück (3) zwischen dem Implantat (2) und einer daran ankoppelbaren extrakorporalen Kopplungseinrichtung aufweist,
**gekennzeichnet durch**,
- eine mit dem Zwischenstück (3) fest verbundene starre Buchse (5) derart, dass zwischen der Wandung der Buchse (5) und dem Zwischenstück (3) ein in Richtung intrakorporal geschlossener Ringraum (6) ausgebildet ist, in den die extrakorporale Kopplungseinrichtung setzbar ist,
- eine offenmaschige, dreidimensionale Raumnetzstruktur (8) auf der Außenwandung der Buchse (5) unter Aussparung im distalen Bereich um eine Breite B, und
- einen Federring (9), der von distal in den Ringraum (6) setzbar, dort teleskopartig verschiebbar und unter Ausübung seiner Federwirkung arretierbar ist.

2. Lager nach Anspruch 1, **dadurch gekennzeichnet, dass** der Federring (9) ein abgewinkelter Ring mit einem Radialschlitz (10) ist.

3. Lager nach Anspruch 1 oder 2, **dadurch gekennzeichnet, dass** der raumnetzstrukturfreie Bereich der Buchse (5) eine Breite B von bis zu 2 cm aufweist.

4. Lager nach einem der Ansprüche 1-3, **dadurch gekennzeichnet, dass** die Maschenweiten der Raumnetzstruktur im Bereich 500-2500 µm betragen.

5. Lager nach einem der Ansprüche 1-4, **dadurch gekennzeichnet, dass** die Buchse (5) durch Aufschrumpfen auf das Zwischenstück (3) fest mit diesem verbunden ist.

6. Lager nach einem der Ansprüche 1-4, **dadurch gekennzeichnet, dass** die Buchse (5), mit dem Zwischenstück verschweißt ist.

7. Lager nach einem der Ansprüche 1-4, **dadurch gekennzeichnet, dass** die Buchse (5) mit dem Zwischenstück (3) einstückig ausgebildet ist.

8. Lager nach einem der Ansprüche 1-7, **dadurch gekennzeichnet, dass** das Zwischenstück (3) ein Doppelkonus mit einem zylindrischen Mittelabschnitt (11) ist, mit welchem die Buchse (5) verbunden ist

9. Subkutanes, intramuskuläres Lager für ein starres transkutanes Implantat (102), welches intrakorporal in einem Knochenstumpf verankerbar ist und welches ein Zwischenstück (103) zwischen dem Implantat (102) und einer daran ankoppelbaren extrakorporalen Kopplungseinrichtung (107) aufweist,
**gekennzeichnet durch**,
eine mit dem Zwischenstück (103) fest verbundene starre Buchse (105) mit einem in Richtung intrakorporal geschlossenen Ankopplungselement (106), an das die extrakorporale Kopplungseinrichtung (107) koppelbar ist,
eine offenmaschige dreidimensionale Raumnetzstruktur (108) auf der Außenwandung der Buchse (105) unter Aussparung im distalen Bereich um eine Breite B,
eine im Bereich des Durchtritts **durch** den Beinstumpf vorgesehene aktivierbare Vorrichtung zur Applikation bioaktiven Materials, und
einen Federring (109), der von distal in das Ankoppelungselement (106) setzbar ist, teleskopartig verschiebbar und unter Ausübung seiner Federwirkung arretierbar ist.

10. Lager nach einem der Ansprüche 2 bis 8 und Anspruch 9,
**dadurch gekennzeichnet, dass** die Vorrichtung umfasst:
mindestens eine in die Außenwandung der Buchse (105) eingebrachte umlaufende Ringnut (110), und
mindestens einen in die Ringnut (110) setzbaren Hohlring (111) aus elastischem und porösem Material mit einem angeformten Zulauf (112), durch welchen der Hohlring (111) mit einem flüssigen bioaktiven Material beschickt werden kann.

11. Lager nach Anspruch 10, **dadurch gekennzeichnet, dass** der Hohlring (111) und der Zulauf (112) aus Silikon bestehen.

12. Lager nach einem der Ansprüche 2 bis 8 und Anspruch 9,
**dadurch gekennzeichnet, dass** die Vorrichtung umfasst:
mindestens eine in die Außenwandung der Buchse (105) eingebrachte umlaufende Ringnut (110), und
mindestens einen seitlich neben dem Ankoppelungselement (106) angeordneten Stichkanal (113) in der Buchse (105), der so verläuft, dass er die mindestens eine Ringnut (110) peripher schneidet, zum Einbringen eines flüssigen bioaktiven Materials.

13. Lager nach Anspruch 12, **dadurch gekennzeichnet, dass** auf der Außenwandung der Buchse (105) im unteren Bereich der Raumnetzstruktur (108) zwischen der untersten Ringnut (110) und dem raumnetzstrukturfreien Bereich der Buchse (105) mindestens ein Stichkanal (114) verläuft zur Weiterleitung des bioaktiven Materials hin zum Durchtritt des Beinstumpfes.

14. Lager nach einem der Ansprüche 2 bis 8 und Anspruch 9, **dadurch gekennzeichnet, dass** um das Ankopplungselement (106) herum ein umlaufender Ringraum (115) angeordnet ist, in welchem ein Vorrat eines elastischen Filmes (116) aus bioaktivem Material gelagert ist, wobei der Film (116) aus dem Ringraum (115) durch einen ringförmigen Schlitz (117) in der Buchse (105) tritt und entlang des raumnetzstrukturfreien Bereichs der Buchse (105) bis zum Durchtritt durch den Beinstumpf, die Hülse (105) schlauchförmig umhüllend, verläuft.

15. Lager nach einem der Ansprüche 1 bis 14, **dadurch gekennzeichnet, dass** das Ankopplungselement (106) als konische Klemmhülse ausgebildet ist.

## Claims

1. Subcutaneous, intramuscular bearing (1) for a rigid transcutaneous implant (2), which can be anchored intracorporeally in a bone stump and which has an intermediate piece (3) between the implant (2) and a extracorporeal coupling device which can be coupled thereto, **characterised by**
- a rigid bushing (5) firmly connected to the intermediate piece (3) such that an annular gap (6) which is closed in the intracorporeal direction is formed between the wall of the bushing (5) and the intermediate piece (3), into which annular gap (6) the extracorporeal coupling device can be placed,
- an open-mesh, three-dimensional spatial network structure (8) on the outer wall of the bushing (5) with exception in the distal region by a width B, and
- a spring ring (9), which can be placed in the annular gap (6) distally, can be displaced there like a telescope and can be locked by exerting its spring action.

2. Bearing according to claim 1, **characterised in that** the spring ring (9) is a bent ring with a radial slot (10).

3. Bearing according to claim 1 or 2, **characterised in that** the region of the bushing (5) which is free of spatial network structure has a width B of up to 2 cm.

4. Bearing according to one of claims 1-3, **characterised in that** the mesh widths of the spatial network structure are in the range 500-2,500 µm.

5. Bearing according to one of claims 1-4, **characterised in that** the bushing (5), by shrinking onto the intermediate piece (3), is firmly connected to the latter.

6. Bearing according to one of claims 1-4, **characterised in that** the bushing (5) is welded to the intermediate piece.

7. Bearing according to one of claims 1-4, **characterised in that** the bushing (5) is designed to be integral with the intermediate piece (3).

8. Bearing according to one of claims 1-7, **characterised in that** the intermediate piece (3) is a double cone with a cylindrical central section (11), to which the bushing (5) is connected.

9. Subcutaneous, intramuscular bearing for a rigid transcutaneous implant (102), which can be anchored intracorporeally in a bone stump and which has an intermediate piece (103) between the implant (102) and a extracorporeal coupling device (107) which can be coupled thereto, **characterised by** a rigid bushing (105) firmly connected to the intermediate piece (103) and having a coupling element (106) which is closed in the intracorporeal direction, to which the extracorporeal coupling device (107) can be coupled, an open-mesh, three-dimensional spatial network structure (108) on the outer wall of the bushing (105) with exception in the distal region by a width B, a device which can be activated and is provided in the region of passage through the leg stump for application of bioactive material, and a spring ring (109), which can be placed in the coupling element (106) distally, can be displaced like a telescope and can be locked by exerting its spring action.

10. Bearing according to one of claims 2 to 8 and claim 9, **characterised in that** the device comprises:
at least one circular annular groove (110) introduced into the outer wall of the bushing (105), and
at least one hollow ring (111), which can be placed in the annular groove (110) and which is made from resilient and porous material having a moulded-on inlet (112), through which the hollow ring (111) may be charged with a liquid bioactive material.

11. Bearing according to claim 10, **characterised in that** the hollow ring (111) and the inlet (112) consist of silicone.

12. Bearing according to one of claims 2 to 8 and claim 9, **characterised in that** the device comprises:
at least one circular annular groove (110) introduced into the outer wall of the bushing (105), and
at least one branch channel (113) in the bushing (105) arranged laterally next to the coupling element (106), which runs so that it intersects the at least one annular groove (110) peripherally, for the introduction of a liquid bioactive material.

13. Bearing according to claim 12, **characterised in that** at least one branch channel (114) runs on the outer wall of the bushing (105) in the lower region of the spatial network structure (108) between the lowermost annular groove (110) and the region of the bushing (105) which is free of spatial network structure, for conveying the bioactive material towards the passage of the leg stump.

14. Bearing according to one of claims 2 to 8 and claim 9, **characterised in that** a circular annular gap (115), in which a supply of a resilient film (116) made from bioactive material is stored, is arranged around the coupling element (106), wherein the film (116) emerges from the annular gap (115) through an annular slot (117) in the bushing (105) and runs along the region of the bushing (105) which is free of spatial network structure as far as the passage through the leg stump, surrounding the sleeve (105) like a tube.

15. Bearing according to one of claims 1 to 14, **characterised in that** the coupling element (106) is designed as a conical clamping sleeve.

## Revendications

1. Support sous-cutané intramusculaire (1) pour un implant transcutané rigide (2), qui peut être ancré par voie intracorporelle dans un moignon d'os et présente une partie intermédiaire (3) entre l'implant (2) et un dispositif de couplage extracorporel qui peut lui être couplé, **caractérisé par**
- une douille rigide (5) raccordée fixement à la partie intermédiaire (3) de manière à former entre la paroi de la douille (5) et la partie intermédiaire (3) un espace annulaire fermé en direction intracorporelle (6), dans lequel on peut disposer le dispositif de couplage extracorporel,
- une structure réticulée tridimensionnelle à mailles ouvertes (8) sur la paroi externe de la douille (5) en formant un évidement dans la zone distale d'une largeur B, et
- une rondelle-ressort (9) qui peut être placée depuis la partie distale dans l'espace annulaire (6), qui peut y être déplacée de manière télescopique et qui peut y être bloquée en exerçant son action élastique.

2. Support selon la revendication 1, **caractérisé en ce que** la rondelle-ressort (9) est un anneau coudé avec une fente radiale (10).

3. Support selon la revendication 1 ou 2, **caractérisé en ce que** la zone de la douille (5) sans structure réticulée présente une largeur B allant jusqu'à 2 cm.

4. Support selon l'une quelconque des revendications 1 à 3, **caractérisé en ce que** les largeurs de mailles de la structure réticulée se situent dans la plage de 500 à 2500 µm.

5. Support selon l'une quelconque des revendications 1 à 4, **caractérisé en ce que** la douille (5) est raccordée solidement à la partie intermédiaire (3) par thermorétraction sur celle-ci.

6. Support selon l'une quelconque des revendications 1 à 4, **caractérisé en ce que** la douille (5) est soudée à la partie intermédiaire.

7. Support selon l'une quelconque des revendications 1 à 4, **caractérisé en ce que** la douille (5) forme un seul tenant avec la partie intermédiaire (3).

8. Support selon l'une quelconque des revendications 1 à 7, **caractérisé en ce que** la partie intermédiaire (3) est un double cône avec une section centrale cylindrique (11) à laquelle la douille (5) est raccordée.

9. Support sous-cutané intramusculaire pour un implant rigide transcutané (102) qui peut être ancré par voie intracorporelle dans un moignon d'os et qui présente une partie intermédiaire (103) entre l'implant (102) et un dispositif de couplage extracorporel qui peut lui être couplé (107), **caractérisé par**
- une douille rigide (105) raccordée fixement à la partie intermédiaire (103) avec un élément de couplage fermé en direction intracorporelle (106) auquel on peut coupler le dispositif de couplage extracorporel (107),
- une structure réticulée tridimensionnelle à mailles ouvertes (108) sur la paroi externe de la douille (105) en ménageant un évidement dans la zone distale d'une largeur B,
- un dispositif activable prévu dans la zone du passage à travers le moignon de jambe pour l'application d'un matériau bioactif, et
- une rondelle-ressort (109) qui peut être placée depuis la partie distale dans l'élément de couplage (106), peut y être déplacée de manière télescopique et peut être bloquée en exerçant son action élastique.

10. Support selon l'une quelconque des revendications 2 à 8 et selon la revendication 9, **caractérisé en ce que** le dispositif comprend :
au moins une rainure annulaire périphérique (110) ménagée dans la paroi externe de la douille (105), et
au moins un anneau creux (111) en matériau élastique et poreux, qui peut être placé dans la rainure annulaire (110) et présente une alimentation moulée (112) par laquelle on peut acheminer un matériau liquide bioactif à l'anneau creux (111).

11. Support selon la revendication 10, **caractérisé en ce que** l'anneau creux (111) et l'alimentation (112) sont constitués de silicone.

12. Support selon l'une quelconque des revendications 2 à 8 et selon la revendication 9, **caractérisé en ce que** le dispositif comprend :
au moins une rainure annulaire périphérique (110) ménagée dans la paroi externe de la douille (105), et
au moins un canal de perçage (113) agencé latéralement à côté de l'élément de couplage (106) dans la douille (105), qui s'étend de manière à couper en périphérie ladite au moins une rainure annulaire (110) pour l'introduction d'un matériau liquide bioactif.

13. Support selon la revendication 12, **caractérisé en ce que**, sur la paroi externe de la douille (105), dans la zone inférieure de la structure réticulée (108) entre la rainure annulaire (110) située le plus bas et la zone de la douille (105) sans structure réticulée, il y a au moins un canal de perçage (114) qui s'étend pour encore acheminer le matériau bioactif afin de lui faire traverser le moignon de jambe.

14. Support selon l'une quelconque des revendications 2 à 8 et selon la revendication 9, **caractérisé en ce qu'**on agence autour de l'élément de couplage (106) un espace annulaire périphérique (115), dans lequel est stockée une réserve de film élastique (116) en matériau bioactif, dans lequel le film (116) sort de l'espace annulaire (115) par une fente annulaire (117) dans la douille (105) et s'étend le long de la zone sans structure réticulée de la douille (105) jusqu'à traverser le moignon de jambe en enveloppant la douille (105) sous forme tubulaire.

15. Support selon l'une quelconque des revendications 1 à 14, **caractérisé en ce que** l'élément de couplage (106) se présente sous la forme d'une douille de serrage conique.
